# EUROPEAN PATENT APPLICATION

(11) **EP 3 831 815 A1**
(43) Date of publication of application: **09.06.2021**
(21) Application number: 20178116.8
(22) Date of filing: 03.06.2020
(51) Int. Cl.: C07D 265/36, C08G 73/02, C09J 161/34

(54) **A BENZOXAZINE ADHESIVE FOR POLYIMIDE AND THE PREPARATION AND APPLICATION METHOD THEREOF**

(30) Priority: 03.12.2019 CN 201911223517
(71) Applicant: Shandong University, Jinan, Shandong 250199 (CN)
(72) Inventor: LU, Haifeng, Jinan, Shandong 250199 (CN); SHAN, Yajuan, Jinan, Shandong 250199 (CN); SUN, Anbang, Jinan, Shandong 250199 (CN); FENG, Shengyu, Jinan, Shandong 250199 (CN); LIU, Shaojie, Jinan, Shandong 250199 (CN); LI, Qiang, Jinan, Shandong 250199 (CN); AN, Chen, Jinan, Shandong 250199 (CN); ZENG, Qingming, Jinan, Shandong 250199 (CN); ZHANG, Di, Jinan, Shandong 250199 (CN)
(74) Representative: Proi World Intellectual Property GmbH

(57) **Abstract**

The invention discloses a benzoxazine adhesive for polyimide and the preparation and application method thereof. The invention, starting from the amino-containing organic compound, can have the phenolic hydroxyl group-containing benzoxazine adhesive prepared through only one step reaction. The preparation method of the invention has simple and controllable synthesis conditions and high synthesis efficiency. The phenolic hydroxyl group-containing benzoxazine adhesive synthesized with the method described in the invention can be used as an adhesive with high adhesion strength for the polyimide material directly; or used as a basic ingredient to produce another adhesive after being added in other fillers so as to meet the different needs of various special occasions. The reaction route of the invention has abandoned the previously reported scheme of using modified polyimide as a surface adhesive for the polyimide material, and has significantly improved the surface adhesion strength of the polyimide material.

## Description

### Technical Field

The invention relates to a benzoxazine adhesive for polyimide and the preparation and application method thereof, and belongs to the field of high polymer material preparation and application.

### Background Art

Polyimide is a kind of organic polymer material with imide ring and heteroaromatic ring constitutional units in the main chain of the molecule. It has strong polarity, stable chemical properties and excellent electrical insulating properties, and is also high and low temperature resistant and radiation resistant. It can be used in a temperature range of 280-450°C. Polyimide based composites have good mechanical and electrical properties, high specific strength and specific stiffness, excellent thermal and chemical stability, a small thermal expansion coefficient, strong solvent resistance, and high dimensional stability, and can be machined into products with complex shapes easily. Therefore, they are widely used in aerospace, electrical and electronic filed, railway transportation and other fields. Thermosetting polyimide is an important variety of polyimide. It becomes soft and flows when heated for the first time, but when heated to a certain temperature, it will have chemical reactions and become harden irreversibly due to cross-link curing. After that, when heated again, it can't go soft or flow again. Thermosetting polyimide resin can be processed with a number of methods, and thus is widely used in aviation, aerospace, electrical appliances, machinery, chemical industry, microelectronics, instrumentation, petrochemical industry, metrology and other high-tech fields. It has become an indispensable material in the world for rocket, aerospace and other cutting-edge technology fields.

Due to poor surface hydrophilicity, thermosetting polyimide materials cannot be adhered to adhesives and metals well. To improve their adhesion, the surface modification treatment method has been used in many studies for the polyimide materials. At present, the relatively mature polyimide surface modification processes include acid and alkali treatment, plasma treatment, ion implantation and surface grafting.

The acid and alkali treatment method is mainly to have the imide groups on the surface of the polyimide material hydrolyzed into polyamide acid and polyamide acid metal salts. Such polar groups can increase the surface energy of the polyimide material, decrease the contact angle and improve the wettability, so the adhesive can evenly infiltrate into the surface of the material, improving the adhesive properties of the material. The treatment liquids that can be used mainly include: sodium hydroxide, potassium hydroxide, potassium permanganate and organic amine solutions, etc.

Plasma is a completely or partially ionized gas-state substance. It contains atoms, molecules, metastable ions and excited ions, as well as roughly the same amount of electrons, positive ions and negative ions. It has higher matter energy and is easy to have physical, chemical and physiological reactions with other substances. According to the discharge modes, low temperature plasmas can be divided into corona discharge, glow discharge, dielectric barrier discharge and radio-frequency discharge, etc. Plasma treatment can have new functional groups (carboxyl, hydroxyl and/or amino groups) formed on the surface of the polyimide material to improve the adhesion, dyeability, printing property, biocompatibility and other properties of the material surface significantly.

Ion beam modification technology is an effective means of controlling the molecular aggregation state with physical methods to realize surface modification. The ion beam surface modification technology can improve not only the surface mechanical properties, but also the optical, electrical, magnetic and adhesive properties of the polymer. The common ion beam modification technologies include reactive oxygen ion beam etching, argon ion beam and nitrogen ion beam irradiation injection modification technology and the like.

The surface grafting method is to treat the surface of the polyimide material first to have active radicals or active centers of functional groups formed, and then triggers grafting polymerization reactions between the monomers and the substrate surface through contact with the monomers relying on the active radicals or the active centers of functional groups on the surface of the material. Plasma treatment on the surface of the polyimide material can introduce polar groups, but the modification effects will decline with time. However, a combination of the plasma treatment and the surface grafting for surface modification can make up for the shortcoming, and create an effective means of enhancing the surface performance of the polyimide material.

The above surface treatment methods all have their own unique advantages, and also their own limitations. For example, the acid and alkali treatment will produce acid and alkali waster liquid, and may cause damages to the metal equipment and hidden safety hazards to the operators; the plasma and ion beam modification technologies, constrained by the size of the operating equipment, cannot be used for surface treatment of large-size polyimide materials; the surface grafting method may cause potential safety hazards due to the use of organic solvents and also cannot handle the surface of large polyimide parts. Therefore, no existing surface treatment method can solve the surface adhesion problem of the polyimide parts.

There are many researches both at home and abroad using materials with low surface energy or polyimide materials as adhesives to adhere the polyimide materials from the perspective of "interface compatibility". For example, Chinese Patent Document No. CN110229609A has disclosed a polyimide coating with functional material; the polyimide coating is prepared by mixing a polyamide acid solution and a functional inorganic filler. When adhesive force of the coating is tested by using a 3M600 adhesive tape, the falling area of the coating does not exceed 10%. Chinese Patent Document No. CN109627764A has disclosed a high-adhesion low-dielectric polyamide film and the preparation method thereof; the invention has presented high adhesion strength by adding fluorinated siloxane grafted modification silicon dioxide pellets into the polyimide. Chinese Patent Document No. CN107722883A has disclosed a modified acrylic bonding sheet adhesive and its binding force with the copper foils or polyimide films is up to 2 kg.

The above technologies have solved the surface adhesion problem of the polyimide material to a certain extent, but the adhesion performance still needs to be further improved.

### Description of the Invention

In view of the poor surface adhesion performance of the polyimide materials of the existing technologies, the invention provides a phenolic hydroxyl group-containing benzoxazine adhesive and the preparation and application method thereof. The adhesive has significant advantages when used for surface adhesion of the polyimide materials. The invention, starting from the amino-containing organic compound, can have the phenolic hydroxyl group-containing benzoxazine adhesive prepared through only one step reaction. The preparation method of the invention has simple and controllable synthesis conditions and high synthesis efficiency. The phenolic hydroxyl group-containing benzoxazine adhesive synthesized with the method described in the invention can be used as an adhesive with high adhesion strength for the polyimide material directly; or used as a basic ingredient to produce another adhesive after being added in other fillers; or used as a primer coating to realize the surface modification of the polyimide material so as to meet the different needs of various special occasions.

### Summary of the Invention

The invention provides a benzoxazine adhesive for polyimide and the preparation and application method thereof. The invention, starting from the amino-containing organic compound, can have the phenolic hydroxyl group-containing benzoxazine adhesive prepared through only one step reaction. The phenolic hydroxyl group-containing benzoxazine adhesive prepared with the method described in the invention can be used as an adhesive directly; or used as a basic ingredient to produce another adhesive after being added in other fillers; or used as a primer coating to meet the different needs of various special occasions.

### Detailed Description of the Invention

The technical solution of the invention is as follows:
A benzoxazine adhesive for polyimide, which is a phenolic hydroxyl group-containing benzoxazine compound
According to a preferred embodiment of the invention, the said adhesive is a compound with the structure as shown in chemical formula (I):

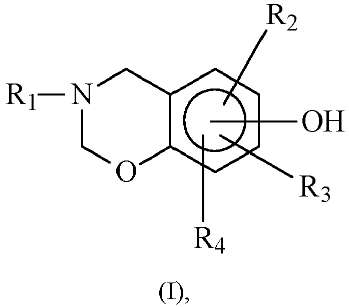

In chemical formula (I), R₁ to R₄ are various organic groups, including various aliphatic hydrocarbon group, silicon alkyl group and/or aromatic hydrocarbon group; or R₂ to R₄ are directly hydrogen atoms respectively, but R₁ cannot be a hydrogen atom.

According to an embodiment of the invention, the preparation method of the said benzoxazine adhesive for polyimide comprises the steps as follows:
Under the conditions of with or without solvents, the amino-containing organic compound is mixed with the aldehyde (ketone) compound and the organic compound with no less than two phenolic hydroxyl groups in a benzene ring to react with each other to get a phenolic hydroxyl group- containing benzoxazine adhesive.

The corresponding reaction equation of the phenolic hydroxyl group-containing benzoxazine adhesive obtained in the invention is as follows:

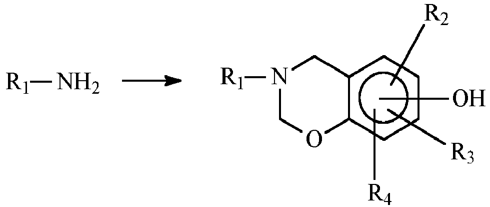

According to a preferred embodiment of the invention, the said amino-containing organic compound is an organic compound containing no less than one amino group, including amino-containing small molecule compound, amino-containing linear polymer, amino-containing comb-like macromolecule, amino-containing dendrimer and amino-containing composite material; according to a further preferred embodiment of the invention, the amino-containing compound is small molecule compound containing multiple amino groups, linear polymer with amino groups in the side chain and amino-containing dendrimer.

According to a preferred embodiment of the invention, the said aldehyde (ketone) compounds are all kinds of aldehyde (ketone) organic compounds that can be dissolved in this system; according to a further preferred embodiment of the invention, the said aldehyde (ketone) compound is small molecule aldehyde (ketone) compound; according to a more preferred embodiment of the invention, the said aldehyde (ketone) compound is formaldehyde, acetone, benzaldehyde or cyclohexanone.

According to a preferred embodiment of the invention, the said organic compound with no less than two phenolic hydroxyl groups in a benzene ring is an organic compound whose molecular structure contains no less than one benzene ring and no less than two phenolic hydroxyl groups in a certain benzene ring; according to a further preferred embodiment of the invention, the said organic compound with no less than two phenolic hydroxyl groups in a benzene ring indicates all kinds of dihydric phenol compounds, trihydric phenol compounds and tetrahydric phenol compounds; according to a more preferred embodiment of the invention, the said organic compound with no less than two phenolic hydroxyl groups in a benzene ring is catechol, resorcinol, hydroquinone, phloroglucinol and four phenolic group benzene.

According to a preferred embodiment of the invention, the said solvents are all kinds of polar or non-polar solvents that can dissolve the reactants, but do not react with the reactants; according to a further preferred embodiment, they are methylbenzene, tetrahydrofuran, chloroform, methyl alcohol, diphenyl ether, dimethyl sulfoxide and N, N-dimethyl formamide; according to a more preferred embodiment, they are methylbenzene and methylbenzene.

According to a preferred embodiment of the invention, the molar ratio of the said amino-containing organic compound, aldehyde (ketone) compound and organic compound with no less than two phenolic hydroxyl groups in a benzene ring is 1: (0.1-15): (0.1-20); according to a further preferred embodiment, the ratio is 1:(2-15):(1-10).

According to a preferred embodiment of the invention, the reaction temperature of the said amino-containing organic compound, aldehyde (ketone) compound and organic compound with no less than two phenolic hydroxyl groups in a benzene ring is 30-180°C, and is further preferred to be 40-80°C.

According to a preferred embodiment of the invention, the reaction time of the said amino-containing organic compound, aldehyde (ketone) compound and organic compound with no less than two phenolic hydroxyl groups in a benzene ring is 1-60h.

According to an embodiment of the invention, a benzoxazine adhesive composition for polyimide comprises the following components in parts by mass:
100 parts of phenolic hydroxyl group-containing benzoxazine adhesive, 0- 10 parts of catalyst, 0-400 parts of filler and 0- 200 parts of auxiliaries.

According to a preferred embodiment of the invention, the said catalyst A is a compound that can catalyze the ring opening and polymerization reaction of the benzoxazine, including the various generalized lewis acids and bases; according to a further preferred embodiment, it is benzenesulfonic acid, acetic acid and sodium hydroxide, and 0-3 parts is preferred to be used.

According to a preferred embodiment of the invention, the said filler is a variety of additives that can improve the performance of the benzoxazinyl adhesive, and is further preferred to be fumed silica, precipitated silica, carbon black, calcium carbonate, aluminium hydroxide and/or aluminium hydroxide as well as any of the above compounds that have been specially treated, and is more preferred to be silazane treated white carbon black. The dosage of the filler is 0- 400 pars and is preferred to be 0-30 parts.

According to a preferred embodiment of the invention, the said auxiliaries are the various auxiliaries that do not significantly reduce the performance of the adhesive after being added, including all kinds of functional components and non-functional components; according to a further preferred embodiment, they are thermal oxygen stabilizer, flame retardant, conductive agent, vesicant, deep curing agent, pigment and/or plasticizer; iron red is further preferred and the dosage is preferred to be 0-10 parts.

According to the invention, the application method of the said benzoxazine adhesive for polyimide comprises the following steps:
The adhesive can be used as an adhesive directly;
Or used together with other catalysts, fillers and auxiliaries.

According to a preferred embodiment of the invention, when used together with other catalysts, fillers and auxiliaries, the phenolic hydroxyl group-containing benzoxazine adhesive is to be mixed with catalyst A, fillers and auxiliaries evenly as a basic ingredient; then, the mixture is to be applied to the adhesive interface to realize good adhesion through programed heating.

According to a preferred embodiment of the invention, the said temperature programming is a process of temperature rising from the initial temperature to the final temperature, with a temperature difference as the gradient and through multiple "temperature rising -constant temperature" links, wherein the said temperature difference is a conditionally controllable temperature gradient allowed by the adhesion conditions, including any temperature difference ranging from 5°C and 100°C, and is preferred to be 20°C; in the said "temperature rising -constant temperature" link, the temperature rising rate is an arbitrary heating rate between 0.1°C/min and 20°C/min, and is preferred to be 10°C/min; in the said "temperature rising -constant temperature" link, the constant temperature time ranges from 1 minute to 180 minutes and is preferred to range from 30 minutes to 120 minutes and more preferred to be 60 minutes; the said initial temperature is an arbitrary temperature value between the room temperature and the final temperature and is preferred to be 60°C; the said final temperature is a temperature value allowed by the adhesion conditions that can achieve the best adhesion performance of the adhesive under such adhesion conditions, including a certain optimum adhesion temperature between 60°C and 200°C obtained through practical experiments.

Steps not specified in details in the invention shall be according to the existing technologies.

The principle and beneficial effects of the invention are as follows:
The reaction route of the invention has combined the hydrogen-bond interaction and the benzoxazine component skillfully. It has realized the preparation of an adhesive with high adhesion strength relying on the no volume shrinkage, high adhesive strength and other characteristics of the benzoxazine during the polymerization and combined with the adhesive properties provided by the hydrogen-bond interaction, and is especially suitable for the surface treatment of the polyimide and other non-stick materials. The reaction route of the invention has abandoned the previously reported scheme of using modified polyimide as a surface adhesive for the polyimide material, and has significantly improved the surface adhesion strength of the polyimide material. Relying on simple and controllable synthesis conditions, high synthesis efficiency and good adhesion properties, the invention has achieved remarkable effects.

The process of the invention comprises one step reaction. Such reaction can occur without the use of any solvent, but the use of solvent is helpful to improve the reaction efficiency. Taking the polarity, boiling point, stability, safety and other aspects of the solvents into consideration, toluene and tetrahydrofuran are preferred. The invention is to introduce the phenolic hydroxyl groups into the structure of the benzoxazine relying on the formation process of the benzoxazine groups. Therefore, to improve the product conversion rate, it is necessary to appropriately increase the feeding ratio of the aldehyde (ketone) compound in this step, depending on the specific type of the aldehyde compound. When formaldehyde solution is used, a 10 times feeding ratio can be selected; when tripolyformaldehyde is used, 2 times feeding ratio can be selected.

The benzoxazine adhesive for polyimide synthesized in the invention can be used directly as an adhesive or as a primer coating, and can also be used to produce other adhesives after compounding with other substances. As the benzoxazine group has the advantage of zero volume contraction during ring-opening copolymerization, such adhesive realizes crosslinking mainly through the ring-opening copolymerization of the benzoxazine groups. Of course, the reactions of other reactive bonds in the system may also have partially participated in the crosslinking. The ring opening process of the benzoxazine groups is generally achieved by heating. In order to ensure that the adhesive has good adhesive property properties, the method of temperature programming is generally used to realize the crosslinking. The temperature programming is a process of temperature rising from the initial temperature to the final temperature, with a temperature difference as the gradient and through multiple "temperature rising -constant temperature" links, wherein the said temperature difference is a conditionally controllable temperature gradient allowed by the adhesion conditions, including any temperature difference ranging from 5°C and 100°C, and is preferred to be 20°C; in the said "temperature rising -constant temperature" link, the temperature rising rate is an arbitrary heating rate between 0.1°C/min and 20°C/min, and is preferred to be 10°C/min; in the said "temperature rising -constant temperature" link, the constant temperature time ranges from 1 minute to 180 minutes and is preferred to range from 30 minutes to 120 minutes and more preferred to be 60 minutes; the said initial temperature is an arbitrary temperature value between the room temperature and the final temperature and is preferred to be 60°C; the said final temperature is a temperature value allowed by the adhesion conditions that can achieve the best adhesion performance of the adhesive under such adhesion conditions, including a certain optimum adhesion temperature between 60°C and 200°Cobtained through practical experiments.

The use of catalyst A can accelerate the ring opening and crosslinking of the benzoxazine groups, reduce the reaction temperature and shorten the reaction time. Both the generalized lewis acids and the generalized lewis bases can promote the ring-opening process. Upon overall consideration, catalyst A is preferred to be benzenesulfonic acid, acetic acid and sodium hydroxide. A too large dosage of the catalyst A will lead to too fast crosslinking reaction, resulting in internal defects of the adhesive system. Therefore, the dosage of the catalyst shall not be excessive. Through comprehensive consideration, the dosage of the catalyst is preferred to be 0- 3 parts.

The benzoxazine adhesive for polyimide prepared in the invention can be used directly as an adhesive. However, as performance of such adhesive is greatly affected by other organic groups, it can only achieve adhesion under specific conditions. The use of various additives (including various fillers and auxiliaries, etc.) to mix with the adhesive can greatly improve the comprehensive performance of the adhesive, so as to meet the adhesion demands of the various occasions. Examples are as follows:
The use of various fillers, especially reinforcement fillers, can improve the mechanical properties of the adhesive greatly. As the base polymer used in the invention contains both benzoxazine groups and phenolic hydroxyl groups, it can be mixed with the various inorganic fillers and organic polar fillers with easy blending, good compatibility, high reinforcement efficiency and other characteristics. In order to further improve the performance of the adhesive at high temperatures, the invention has further preferred the white carbon black treated with the phenyl group containing compounds for reinforcement, so as to slow down the softening and thermal degradation phenomena of the adhesive when used at high temperatures.

The addition of various auxiliaries, such as thermal oxygen stabilizer, flame retardant, conductive agent, vesicant, deep curing agent, pigment and plasticizer, to the formula involved in the invention can further improve the performance of the adhesive and expand its applications. Certain auxiliaries can also play a number of roles. For example, the iron red can serve as hot oxygen stabilizer, pigment and reinforcement filler at the same time. The addition of special polymers can improve the wettability of such kind of adhesives and specific interfaces. The addition of special coupling agents can improve the adhesive performance of this kind of adhesives and special interfaces. In short, by changing the dosage of additives and using a variety of additives together, a high performance adhesive can be produced to meet the needs of various occasions, with broad application prospects and good market prospects.

### Brief Description of the Figures

Figure 1 is the h-nuclear magnetic resonance spectrum of the raw material G1 used in the benzoxazine adhesive obtained in Embodiment 2.
Figure 2 is the infrared spectrum of the benzoxazine adhesive with phenolic hydroxyl containing benzoxazine groups at the two ends obtained in Embodiment 2.
Figure 3 is the nuclear magnetic resonance spectrum of the benzoxazine adhesive with phenolic hydroxyl containing benzoxazine groups at the two ends obtained in Embodiment 2.

### Detailed Embodiments

The invention is further described as follows in combination with the specific embodiments, but is not limited to that.

The raw materials used in the embodiments are all conventional raw materials available on the market or can be prepared according to the methods in the references.

The molar ratios as described in the embodiments are proportions of the amount of substances. All proportions are expressed as parts by mass.

### Embodiment 1

Aminopropyl-terminated disiloxane, phloroglucinol and trioxymethylene are blended evenly in methylbenzene according to the molar ratio 1:10:4; the temperature is maintained at 80°C and the mixture is mechanically agitated for 6 hours; the phenolic hydroxyl group-containing benzoxazine adhesive is then obtained upon purification with a reaction yield of 85%.

### Embodiment 2

As described in Embodiment 1, provided that the aminopropyl-terminated disiloxane is changed to silicon-containing dendrimer with amino groups as the end groups (G1), the trioxymethylene is changed to formaldehyde solution (37wt%), the phloroglucinol is changed to catechol, and their molar ratio is changed to 1:2:4; the reaction temperature is changed to 70°C; the mechanical agitation time is changed to 5 hours; and the benzoxazine adhesive with phenolic hydroxyl group-containing benzoxazine groups at the two ends is obtained, with a reaction yield of 97%.

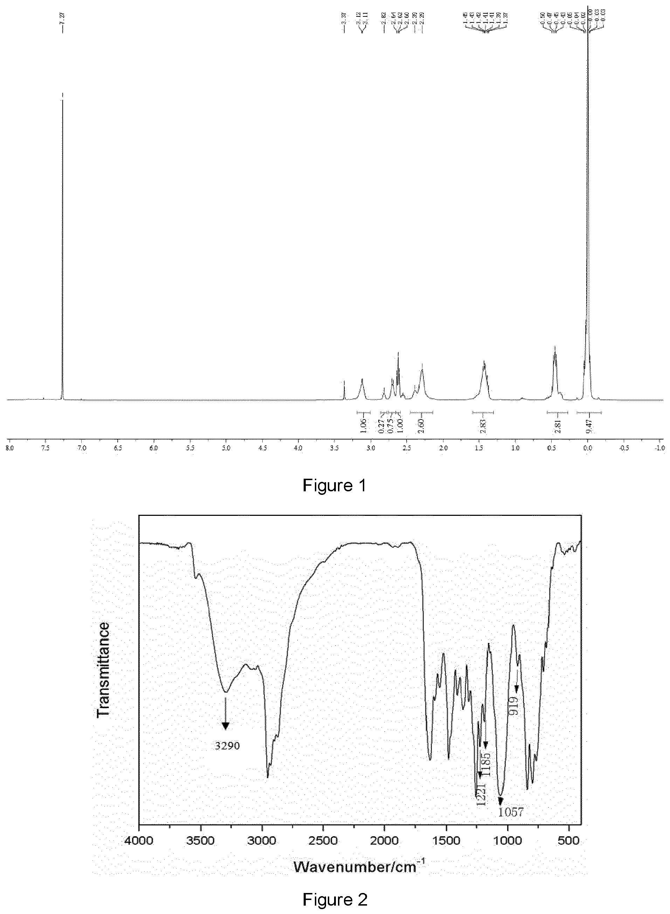

The h-nuclear magnetic resonance spectrum of the raw material G1 used in the benzoxazine adhesive is as shown in figure 1: ¹H NMR (CDCl₃): δ (ppm) 0.01 (s, 60H, SiCH₃), 0.45 (t, 20H, SiCH₂CH₂CH₂N), 1.34- 1.44 (m, 20H, SiCH₂CH₂CH₂N), 2.30 (t, 8H, CH₂CONH), 2.42 (t, 4H, SiCH₂CH₂CH₂N), 2.65 (t, 8H, SiCH₂CH₂CH₂NH₂), 2.74 (t, 8H, NCH₂CH₂CONH), 3.15 (t, 8H, SiCH₂CH₂CH₂NHCO).

IR characterization of the synthesized phenolic hydroxyl group-containing benzoxazine adhesive is as shown in Figure 2. As can be seen from the Figure 2, the peak at 1057cm⁻¹ is generated by the asymmetric bending and vibration of the Si-O-Si. The peaks at 1185 and 1221cm⁻¹ are respectively the asymmetric stretching vibration peaks of the C-O-C and C-N-C on the oxazine ring. The peak at 919cm⁻¹ is the characteristic absorption peak of the benzene ring connected to the oxazine ring, namely a peak of the said oxazine ring, the absorption peak at 1482cm⁻¹ is a stretching vibration peak on the triple-substitution benzene ring, and the bulge peak at 3290cm⁻¹ is resulted in by the hydrogen bonds formed by the hydroxyl groups on the benzene ring.

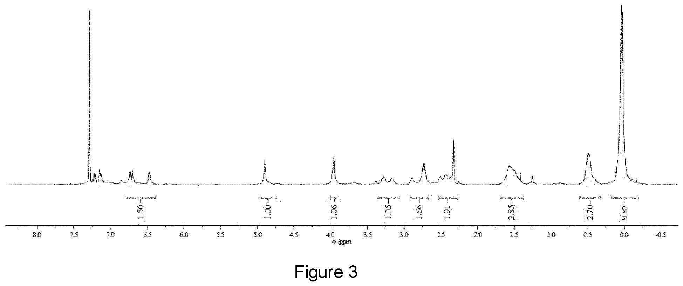

NMR characterization of the synthesized phenolic hydroxyl group-containing benzoxazine adhesive is as shown in Figure 3: ¹ HNMR (CDCl₃): δ (ppm) 0.01 (s, 60H, SiCH₃), 0.45 (t, 20H, SiCH₂CH₂CH₂N), 1.34- 1.44 (m, 20H, SiCH₂CH₂CH₂N), 2.30 (t, 8H, CH₂CONH), 2.42 (t, 4H, SiCH₂CH₂CH₂N), 2.74 (t, 8H, NCH₂CH₂CONH), 3.15 (t, 8H, SiCH₂CH₂CH₂NHCO),δ (ppm) 3.94 (s, 8H, O-CH₂-N),δ (ppm) 4.93(s, 8H,Ar-CH₂-N),δ (ppm) 6-7(m, 12H,Ar-H).

The reaction process of the benzoxazine adhesive obtained in the embodiment is as follows:

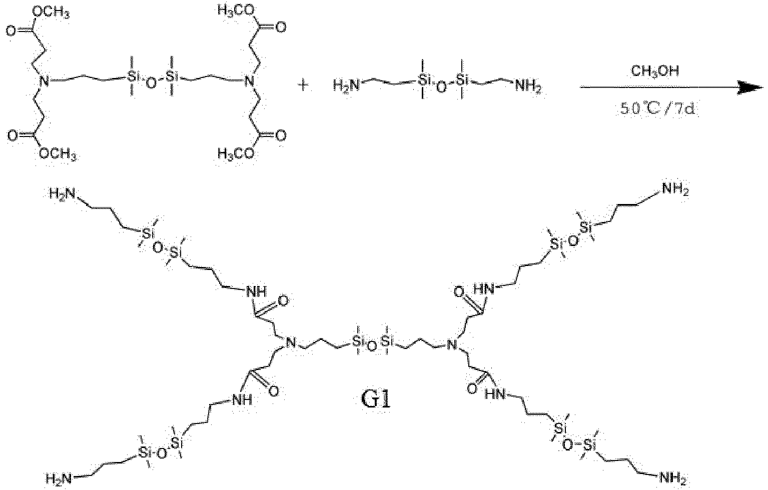

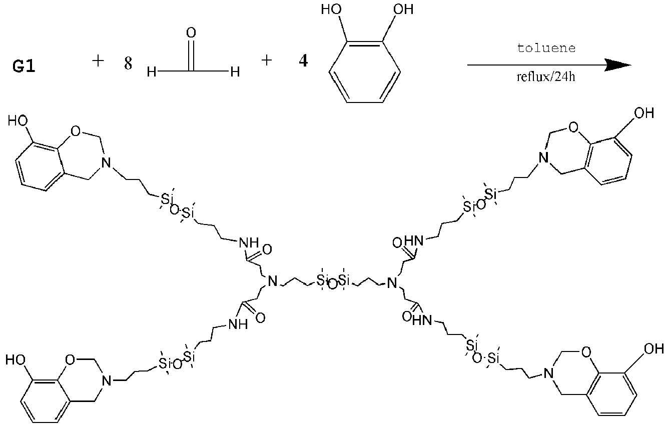

### Embodiment 3

As described in Embodiment 2, provided that the silicon-containing dendrimer with amino groups as the end groups is changed to ethylenediamine, while the other parameters are maintained the same; and the benzoxazine adhesive with phenolic hydroxyl group-containing benzoxazine groups at the two ends is obtained, with a reaction yield of 100%.

### Embodiment 4

As described in Embodiment 3, provided that the ethylenediamine is changed to aminopropyl-terminated polysiloxane (number-average molar mass 1000); and the benzoxazine adhesive of a polysiloxane containing structure with phenolic hydroxyl group-containing benzoxazine groups at the two ends is obtained, with a reaction yield of 100%.

### Embodiment 5

As described in Embodiment 3, provided that the ethylenediamine is changed to polysiloxane with aminopropyl groups in the side chain (aminopropyl unit content in the polysiloxane: 5%; number-average molar mass: 120000); and finally, the polysiloxane with benzoxazine groups in the side chain is obtained, with a reaction yield of 90%.

### Embodiment 6

As described in Embodiment 3, provided that the ethylenediamine is changed to hexanediamine; and the benzoxazine adhesive with phenolic hydroxyl group-containing benzoxazine groups at the two ends of the molecule is obtained, with a reaction yield of 96%.

### Embodiment 7

As described in Embodiment 6, provided that the hexanediamine is changed to aminopropyl trimethoxy silane; and the benzoxazine adhesive with a phenolic hydroxyl group-containing benzoxazine group at one end is obtained, with a reaction yield of 96%.

### Embodiment 8

As described in Embodiment 7, provided that the aminopropyl trimethoxy silane is changed to aminopropyl vinyl diethoxy silane; and the benzoxazine adhesive with a vinyl diethoxy silane-containing benzoxazine group at one end is obtained, with a reaction yield of 98%.

### Embodiment 9

As described in Embodiment 8, provided that the aminopropyl vinyl diethoxy silane is changed to aminopropyl dimethyl ethoxy silane; the formaldehyde solution is changed to p-hydroxy dibenzophenone; the solvent is changed to tetrahydrofuran; and the dimethyl ethoxy silane with a phenolic hydroxyl group-containing benzoxazine group at one end is obtained, with a reaction yield of 95%.

### Embodiment 10

As described in Embodiment 8, provided that the aminopropyl vinyl diethoxy silane is changed to aminopropyl dimethyl ethoxy silane; the p-hydroxy benzophenone is changed to p-hydroxy phenyl ethyl ketone; the solvent is changed to methylbenzene; and the dimethyl ethoxy silane with a phenolic hydroxyl group-containing benzoxazine group at one end is obtained, with a reaction yield of 93%.

### Embodiment 11

As described in Embodiment 8, provided that the aminopropyl vinyl diethoxy silane is changed to aminopropyl triethoxy silane; the p-hydroxy benzophenone is changed to o-hydroxybenzaldehyde; the solvent is changed to chloroform; and the benzoxazine group-containing triethoxysilane is obtained, with a reaction yield of 96%.

### Embodiment 12

As described in Embodiment 3, provided that acetic acid is added as the catalyst for the first step reaction and the dosage is 5% of the amount of ethylenediamine; the reaction time is changed to 3h; and the benzoxazine adhesive with benzoxazine groups at the two ends is obtained, with a reaction yield of 94%.

### Embodiment 13

The benzoxazine adhesive of a siloxysilane structure with phenolic hydroxyl group-containing benzoxazine groups at the two ends obtained in Embodiment 2 is applied to the polyimide plate, and then is cured under certain conditions (curing conditions: heating at 80°C for 1h; heating at 90°C for 1h; heating at 100°C for 1h; heating at 110°C for 1h; and heating at 120°C for 1h). The plate is kept still for 1 day after curing, and then is to be tested with a tensile machine.

### Embodiment 14

The benzoxazine adhesive of a siloxysilane structure with phenolic hydroxyl group-containing benzoxazine groups at the two ends obtained in Embodiment 2 is used as a basic formula component (100 parts) and then is mixed evenly with fumed silica (5 parts) and iron red (1 part); the mixture is then applied evenly to the polyimide plate, and is cured under certain conditions (curing conditions: heating at 80°C for 1h; heating at 90°C for 1h; heating at 100°C for 1h; heating at 110°C for 1h; and heating at 120°C for 1h). The plate is kept still for 1 day after curing, and then is to be tested with a tensile machine.

### Embodiment 15

The benzoxazine adhesive with phenolic hydroxyl group-containing benzoxazine groups at the two ends obtained in Embodiment 3 is applied to the polyimide plate, and then is cured under certain conditions (curing conditions: heating at 120°C for 1h; heating at 130°C for 1h; heating at 140°C for 1h; heating at 150°C for 1h; and heating at 160°C for 1h). The plate is kept still for 1 day after curing, and then is to be tested with a tensile machine.

### Embodiment 16

The benzoxazine adhesive with phenolic hydroxyl group-containing benzoxazine groups at the two ends obtained in Embodiment 3 is used as a basic formula component and then is mixed evenly with acetic acid, M-5 white carbon black treated with silazane (15 parts) and silane coupling agent KH570 (3 parts); the mixture is then applied evenly to the steel plate, and is cured under certain conditions (curing conditions: heating at 90°C for 1h; heating at 100°C for 1h; heating at 110°C for 1h; heating at 120°C for 1h; and heating at 130°C for 1h). The plate is kept still for 1 day after curing, and then is to be tested with a tensile machine.

### Experimental Case 1

Adhesive strength data of the embodiments tested are as shown in Table 1.

**Table 1**

| S/N | Adhesive strength, MPa | | | |
|---|---|---|---|---|
| | 1 | 2 | 3 | Average |
| Embodiment 14 | Plate fracture*** | Plate fracture*** | Plate fracture*** | Plate fracture*** |
| Embodiment 15 | Plate fracture*** | Plate fracture*** | Plate fracture*** | Plate fracture*** |
| Embodiment 16 | Plate fracture*** | Plate fracture*** | Plate fracture*** | Plate fracture*** |
| Comparative case 1* | - | - | - | 4.3 |
| Comparative case 2* | - | - | - | 5 |

| | | | | |
|---|---|---|---|---|
| *Note: Data of comparative case 1 come from the patent (publication number: CN109880541A). **Note: Data of comparative case 2 come from the patent (publication number: CN108641665A). ***Note: Plate fracture in the table indicates fracture of the PI plates bonded together, and the average tensile force at the plate fracture can reach up to 3.18MPa. | | | | |

As can be seen from Table 1, the phenolic hydroxyl group-containing benzoxazine adhesive prepared in the invention can be used as an adhesive with high strength directly. When the phenolic hydroxyl group-containing benzoxazine adhesive is mixed with other fillers and auxiliaries, its performance is still very good, and its overall adhesion strength is even higher than the tensile strength data of the polyimide material coatings reported in the literatures. Such data show that the adhesive in the invention has obvious superiority in performance. Combined with the advantages of the invention in the preparation process, the creativity of the invention is further demonstrated.

## Claims

1. A benzoxazine adhesive for polyimide, **characterized in that** the adhesive is a phenolic hydroxyl group-containing benzoxazine compound.

2. The benzoxazine adhesive for polyimide according to Claim 1, **characterized in that** the said adhesive is a compound with the structure as shown in chemical formula (I): 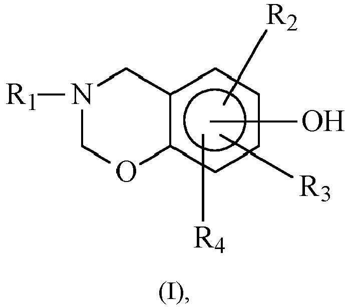 In chemical formula (I), R₁ to R₄ are various organic groups, including various aliphatic hydrocarbon groups, silicon alkyl groups and/or aromatic hydrocarbon groups; or R₂ to R₄ are directly hydrogen atoms respectively, but R₁ cannot be a hydrogen atom.

3. The preparation method of the said benzoxazine adhesive for polyimide according to Claim 1 comprises the steps as follows:
Under the conditions of with or without solvents, the amino-containing organic compound is mixed with the aldehyde (ketone) compound and the organic compound with no less than two phenolic hydroxyl groups in a benzene ring to react with each other to get a phenolic hydroxyl group- containing benzoxazine adhesive.

4. The said preparation method of the benzoxazine adhesive for polyimide according to Claim 3, **characterized in that** the said amino-containing organic compound is an organic compound containing no less than one amino group.

5. The said preparation method of the benzoxazine adhesive for polyimide according to Claim 3, **characterized in that** the said amino-containing organic compound includes amino-containing small molecule compound, amino-containing linear polymer, amino-containing comb-like macromolecule, amino-containing dendrimer and amino-containing composite material;
According to a further preferred embodiment of the invention, the amino-containing compound is small molecule compound containing multiple amino groups, linear polymer with amino groups in the side chain and amino-containing dendrimer.

6. The said preparation method of the benzoxazine adhesive for polyimide according to Claim 3, **characterized in that** the said aldehyde (ketone) compounds are all kinds of aldehyde (ketone) organic compounds that can be dissolved in this system.

7. The said preparation method of the benzoxazine adhesive for polyimide according to Claim 3, **characterized in that** the said aldehyde (ketone) compound is formaldehyde, acetone, benzaldehyde or cyclohexanone.

8. The said preparation method of the benzoxazine adhesive for polyimide according to Claim 3, **characterized in that** the said organic compound with no less than two phenolic hydroxyl groups in a benzene ring is an organic compound whose molecular structure contains no less than one benzene ring and no less than two phenolic hydroxyl groups in a certain benzene ring.

9. The said preparation method of the benzoxazine adhesive for polyimide according to Claim 3, **characterized in that** the said organic compound with no less than two phenolic hydroxyl groups in a benzene ring indicates all kinds of dihydric phenol compounds, trihydric phenol compounds and tetrahydric phenol compounds, and are preferred to be catechol, resorcinol, hydroquinone, phloroglucinol and four phenolic group benzene.

10. The said preparation method of the benzoxazine adhesive for polyimide according to Claim 3, **characterized in that** the said solvents are all kinds of polar or non-polar solvents that can dissolve the reactants, but do not react with the reactants, and are preferred to be methylbenzene, tetrahydrofuran, chloroform, methyl alcohol, diphenyl ether, dimethyl sulfoxide and N, N-dimethyl formamide.

11. The said preparation method of the benzoxazine adhesive for polyimide according to Claim 3, **characterized in that** the molar ratio of the said amino-containing organic compound, aldehyde (ketone) compound and organic compound with no less than two phenolic hydroxyl groups in a benzene ring is 1: (0.1-15): (0.1-20) and is further preferred to be 1:(2-15):(1-10).

12. The said preparation method of the benzoxazine adhesive for polyimide according to Claim 3, **characterized in that** the reaction temperature of the said amino-containing organic compound, aldehyde (ketone) compound and organic compound with no less than two phenolic hydroxyl groups in a benzene ring is 30-180°C, and is further preferred to be 40-80°C.

13. A benzoxazine adhesive composition for polyimide, **characterized in that** the composition comprises the following components in parts by mass:
100 parts of phenolic hydroxyl group-containing benzoxazine adhesive, 0- 10 parts of catalyst, 0-400 parts of filler and 0- 200 parts of auxiliaries.

14. The said benzoxazine adhesive composition for polyimide according to Claim 13, **characterized in that** the said catalyst A is a compound that can catalyze the ring opening and polymerization reaction of the benzoxazine, and is preferred to be benzenesulfonic acid, acetic acid and sodium hydroxide;
The said filler is a variety of additives that can improve the performance of the benzoxazinyl adhesive, and is further preferred to be fumed silica, precipitated silica, carbon black, calcium carbonate, aluminium hydroxide and/or aluminium hydroxide;
The said auxiliaries are the various auxiliaries that do not significantly reduce the performance of the adhesive after being added, and are preferred to be thermal oxygen stabilizer, flame retardant, conductive agent, vesicant, deep curing agent, pigment and/or plasticizer.

15. The application method of the said benzoxazine adhesive for polyimide according to Claim 1 comprises the following steps:
The adhesive can be used as an adhesive directly;
Or used together with other catalysts, fillers and auxiliaries.
